# EUROPEAN PATENT APPLICATION

(11) **EP 4 840 848 A1**
(43) Date of publication of application: **18.11.2026**
(21) Application number: 25175816.5
(22) Date of filing: 12.05.2025
(51) Int. Cl.: C07D 209/16, A61P 25/00, A61P 25/24, A61K 31/4045

(54) **NEW PSILOCIN DERIVATIVE PRODRUGS**

(71) Applicant: Preseljeno & Regulirano d.o.o., 80101 Livno (BA)
(72) Inventor: STAUDT, Marek, 63486 Bruchköbel (DE); GAWOR, Yannik, 63486 Bruchköbel (DE)
(74) Representative: Patentanwälte Olbricht Buchhold Keulertz

(57) **Abstract**

The present invention relates to new prodrugs of tryptamine represented by Formula (I), and a method of preparation thereof. In addition, the invention pertains to their use as a medicament, in particular, in the medical and therapeutic treatment of mental health disorders.

## Description

### Field of the invention

The present invention relates to new prodrugs of tryptamine, and a method of preparation thereof. In addition, the invention pertains to their use as a medicament, in particular, in the medical and therapeutic treatment of mental health disorders.

### Background of the invention

Mental health disorders such as major depressive disorder (MDD), treatment-resistant depression, postpartum depression, and post-traumatic stress disorder (PTSD) constitute major global health challenges. Additional conditions such as generalized anxiety disorder, unipolar and bipolar depressive disorders, and other forms of depressive illness further contribute to the global disease burden. Conventional treatments, including SSRIs, often show delayed onset, limited efficacy, and adverse side effects, highlighting the need for faster-acting, more effective, and better-tolerated therapies.

Recent developments have revived interest in serotonergic psychedelics for treating mood and anxiety disorders. Compounds like psilocybin and LSD have shown rapid, sustained benefits in clinical trials, especially for patients with major depression, treatment-resistant forms of depression, or trauma-related psychiatric conditions. These effects are largely attributed to 5-HT2A receptor agonism and modulation of neuroplasticity, emotional regulation, and cognitive flexibility.

4-hydroxy-N-methyl-N-ethyltryptamine (4-HO-MET), a synthetic tryptamine structurally related to psilocin, has shown promising therapeutic potential in preliminary studies. It offers advantages such as a shorter duration and milder psychoactive profile, which may enhance clinical practicality and reduce psychological burden, particularly in individuals with anxiety-related disorders, PTSD, and various depressive subtypes.

Unlike psilocybin, 4-HO-MET is fully synthetic, enabling improved control over purity, scalability, and standardization - key factors in pharmaceutical development. However, its clinical utility is limited by suboptimal pharmacokinetic properties, including rapid metabolism and low oral bioavailability.

It is hence an object of the present invention to provide a suitable pro-drug of 4-HO-MET which overcomes the downsides of established 4-HO-MET prodrugs.

### Summary of the invention

The technical problem described above is solved by the present invention.

To overcome the problems, in a first aspect, this invention provides a prodrug of 4-HO-MET according to Formula (I) designed to improve pharmacokinetic performance, enhance oral bioavailability, and enable a faster and more predictable onset of therapeutic action. This novel formulation facilitates the safe and effective treatment of a broad range of psychiatric disorders, including unipolar and bipolar depression, generalized anxiety disorder, major depression, treatment-resistant depression, and postpartum depression, representing a significant advancement in psychedelic-assisted psychotherapy.

**In** a second aspect, the present invention is directed to the use of the compound according to Formula (I) for use as a medicament.

**In** a further aspect, the present invention refers to a pharmaceutical composition comprising a compound of Formula (I).

In a final aspect, the present invention is also directed to a method for preparing a compound according to Formula (I).

### Description of the invention

The present invention relates to a compound represented by Formula (I), a pharmaceutically acceptable salt, a solvate, and/or hydrate thereof:

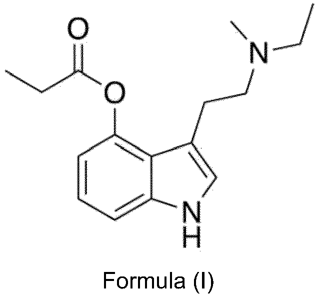

The salt can be selected from the group consisting of benzoate, fumarate, malate, maleate, lactate, salicylate, tartrate, gluconate, pamoate, stearate, camphorsulfonate, methanesulfonate, ethanesulfonate, benzenesulfonate, tosylate, nitrate, nitrite, citrate, hydrogen citrate, dihydrogen citrate, acetate, propionate, butyrate, valerate, succinate, phosphate, hydrogen phosphate, dihydrogen phosphate, sulfate, hydrogen sulfate, hydrofluoride, hydrochloride, hydrobromide, hydroiodide, oxalate, malonate, triflate, glutamate, ascorbate, glycolate, mandelate, isethionate, nicotinate, hippurate, laurate and pantothenate salt. Preferably, the salt is selected from the group consisting of oxalate, fumarate and succinate.

The compound represented by Formula (I) can also be used as a medicament. Preferably, the compound represented by Formula (I) can be used in the treatment of mental disorder such as a depressive condition, including unipolar and bipolar depressive conditions, depression from generalized anxiety, major depression, treatment resistant depression and postpartum depression.

The present invention is also directed to a composition comprising a compound represented by Formula (I).

The compound and the composition according to the present invention can be administered orally.

The present invention is also related to a method for preparing the compound represented by Formula (I). The method comprises the steps a. to e.:
In step a, an organic solution comprising 4-acetoxyindole is provided. Preferably, Et₂O is used as solvent.

In step b, 4-acetoxyindole is reacted with oxalyl chloride to obtain 3-(2-chloro-2-oxoacetyl)-1H-indol-4-yl acetate.

In the next step c, ethylmethylamine is reacted with the 3-(2-chloro-2-oxoacetyl)-1H-indol-4-yl acetate to obtain the 3-(2-(ethyl(methyl)amino)-2-oxoacetyl)-1H-indol-4-yl acetate.

In step d, the obtained 3-(2-(ethyl(methyl)amino)-2-oxoacetyl)-1H-indol-4-yl acetate is reduced with a reducing agent to obtain 4-hydroxy-N-methyl-N-ethyltryptamine (4-HO-MET). As reducing agent LAH is preferably employed.

In step e, the obtained 4-HO-MET is reacted with propionic anhydride to obtain 4-propionoxy-N-methyl-N-ethyltryptamine (4-Pro-MET).

The steps of the method are conducted in the order a., b., c., d., and then e.

In an embodiment of the present invention, the method also comprises step f, wherein the 4-Pro-MET is reacted with a compound selected from the group consisting of benzoic acid, fumaric acid, malic acid, maleic acid, lactic acid, salicylic acid, tartaric acid, gluconic acid, pamoic acid, stearic acid, camphorsulfonic acid, methanesulfonic acid, ethanesulfonic acid, benzenesulfonic acid, toluene sulfonic acid, nitric acid, citric acid, acetic acid, propionic acid, butanoic acid, pentanoic acid, succinic acid, phosphor acid, sulfuric acid, hydrogen fluoride, hydrogen chloride, hydrogen bromide, hydrogen iodide, oxalic acid, malonic acid, trifluoromethanesulfonic acid, glutamic acid, ascorbic acid, glycolic acid, mandelic acid, isethionic acid, nicotinic acid, hippuric acid, lauric and pantothenic acid. Preferably, oxalic acid, fumaric acid or succinic acid is employed. By doing so, a pharmaceutical acceptable salt of the compound of Formula (I) according to the invention is obtained.

### Examples

### Example 1: 4-Pro-MET fumarate

1 kg of 4-acetoxyindole was dissolved in 10 L of Et₂O and cooled to 0 °C. To this mixture, 1 L of oxalyl chloride was added dropwise making sure that the temperature did not exceed a temperature of 5°C. Subsequently, the mixture was stirred for an additional 30 minutes, centrifuged, charged back in the reactor and dissolved in 20 L of 2-MeTHF. Ethylmethylamine was added until a pH value of 10 was achieved. The resulting mixture was stirred for 30 minutes and then washed twice with 10 L of a 0.1M HCI. The solvent was stripped to yield 1.2 kg of the glyoxylamide. 12.5 L of 2-MeTHF was charged to the reactor to dissolve gloxylamide and discharged into a separate container. 12.5 L fresh 2-MeTHF was added to the reactor. 660 g LAH was added slowly while stirring. After 30 minutes, the previously prepared solution was added dropwise slowly over a 30-minute period then set to reflux for 5 hours. The solution was cooled overnight, quenched with 10 L of water, centrifuged, and washed with 10 L of water. The organic layer was stripped, and 4-HO-MET was vacuum distilled. The distillate was redissolved in 10 L DCM, to this 0.2 L pyridine and 2 L propanoic anhydride was added. The solution was stirred for 30 minutes, washed with water three times and then stripped under vacuum to yield the 4-PrO-MET freebase. The latter was redissolved in 30 L acetone and fumaric acid added up to a pH value of 4 was achieved. The solution was stirred for an additional 30 minutes and then centrifuged to yield 0.84 kg of 4-propionoxy-N-methyl-N-ethyltryptamine fumarate.

¹H-NMR (400 MHz; DMSO-d6): δ = 11.15 (s, br, 1H), 7.24 (d, J = 7.6 Hz, 1H), 7.20 (d, J = 2.4 Hz, 1H), 7.05 (t, J = 8.0 Hz, 1H), 6.68 (d, J = 7.2 Hz, 1H), 6.53 (s, 2H), 2.94 (s, 4H), 2.82 (q, J = 7.2 Hz, 2H), 2.73 (q, J = 7.2 Hz, 2H), 2.52 (s, 3H), 1.18 (t, J = 7.6 Hz, 3H), 1.12 (t, J = 7.2 Hz, 3H).

## Claims

1. A compound represented by Formula (I), a pharmaceutically acceptable salt, a solvate, and/or hydrate thereof:

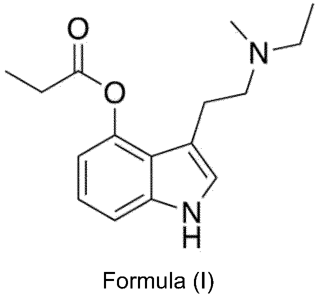

2. The compound according to claim 1, **characterized in that** the salt is selected from the group consisting of benzoate, fumarate, malate, maleate, lactate, salicylate, tartrate, gluconate, pamoate, stearate, camphorsulfonate, methanesulfonate, ethanesulfonate, benzenesulfonate, tosylate, nitrate, nitrite, citrate, hydrogen citrate, dihydrogen citrate, acetate, propionate, butyrate, valerate, succinate, phosphate, hydrogen phosphate, dihydrogen phosphate, sulfate, hydrogen sulfate, hydrofluoride, hydrochloride, hydrobromide, hydroiodide, oxalate, malonate, triflate, glutamate, ascorbate, glycolate, mandelate, isethionate, nicotinate, hippurate, laurate and pantothenate salt.

3. The compound according to claim 1 or 2 for use as a medicament.

4. The compound according to claim 1 or 2 for use in the treatment of mental disorder such as a depressive condition, including unipolar and bipolar depressive conditions, depression from generalized anxiety, major depression, treatment resistant depression and postpartum depression.

5. A pharmaceutical composition comprising the compound according to claim 1 or 2.

6. A method for preparing the compound represented by Formula (I) comprising the steps of:
a. Providing an organic solution comprising 4-acetoxyindole,
b. Reacting the 4-acetoxyindole with oxalyl chloride to obtain 3-(2-chloro-2-oxoacetyl)-1H-indol-4-yl acetate,
c. Reacting ethylmethylamine with 3-(2-chloro-2-oxoacetyl)-1H-indol-4-yl acetate to obtain 3-(2-(ethyl(methyl)amino)-2-oxoacetyl)-1H-indol-4-yl acetate,
d. Reducing 3-(2-(ethyl(methyl)amino)-2-oxoacetyl)-1H-indol-4-yl acetate with a reducing agent to obtain 4-hydroxy-N-methyl-N-ethyltryptamine, and
e. Reacting 4-hydroxy-N-methyl-N-ethyltryptamine with propanoic anhydride to obtain 4-propionoxy-N-methyl-N-ethyltryptamine.

7. Method according to claim 6 comprising the step of:
f. Reacting the 4-propionioxy-N-methyl-N-ethyltryptamine obtained in step e. with a compound selected from the group consisting of benzoic acid, fumaric acid, malic acid, maleic acid, lactic acid, salicylic acid, tartaric acid, gluconic acid, pamoic acid, stearic acid, camphorsulfonic acid, methanesulfonic acid, ethanesulfonic acid, benzenesulfonic acid, toluene sulfonic acid, nitric acid, citric acid, acetic acid, propionic acid, butanoic acid, pentanoic acid, succinic acid, phosphor acid, sulfuric acid, hydrogen fluoride, hydrogen chloride, hydrogen bromide, hydrogen iodide, oxalic acid, malonic acid, trifluoromethanesulfonic acid, glutamic acid, ascorbic acid, glycolic acid, mandelic acid, isethionic acid, nicotinic acid, hippuric acid, lauric and pantothenic acid.
